# EUROPÄISCHE PATENTSCHRIFT

(11) **EP 0 797 571 B1**
(45) Veröffentlichungstag und Bekanntmachung des Hinweises auf die Patenterteilung: **24.03.1999**
(21) Anmeldenummer: 95941645.4
(22) Anmeldetag: 01.12.1995
(51) Int. Cl.: C07D 201/12, C08J 11/14

(54) **VERFAHREN ZUR GEWINNUNG VON CAPROLACTAM DURCH HYDROLYTISCHE SPALTUNG VON GESCHMOLZENEM POLYCAPROLACTAM**
METHOD OF OBTAINING CAPROLACTAM BY HYDROLYTIC SPLITTING OF MOLTEN POLYCAPROLACTAM
PROCEDE D'OBTENTION DE CAPROLACTAME PAR DIVISION HYDROLYTIQUE DE POLYCAPROLACTAME FONDU

(30) Priorität: 12.12.1994 US 355286
(43) Veröffentlichungstag der Anmeldung: 01.10.1997
(73) Patentinhaber: BASF AKTIENGESELLSCHAFT, 67056 Ludwigshafen (DE)
(72) Erfinder: BASSLER, Peter, D-68519 Viernheim (DE); KOPIETZ, Michael, D-67269 Grünstadt (DE)
(86) Internationale Anmeldenummer: EP9504740
(87) Internationale Veröffentlichungsnummer: WO9618613

(56) Entgegenhaltungen:
- EP-A- 0 612 727
- EP-A- 0 676 394
- WO-A-94/06763
- US-A- 3 939 153
- US-A- 4 605 762

## Beschreibung

Die vorliegende Erfindung betrifft ein verbessertes Verfahren zur Gewinnung von Caprolactam aus caprolactamhaltigen Polymeren in Gegenwart von überhitztem Wasser.

Des weiteren betrifft die Erfindung eine Vorrichtung zur Durchführung des erfindungsgemäßen Verfahrens sowie die Verwendung des erfindungsgemäßen Verfahrens und der erfindungsgemäßen Vorrichtung zum Recycling von Polycaprolactam-haltigen Abfällen.

In der US 4,605,762 wird ein kontinuierliches Verfahren zur hydrolytischen Depolymerisation von Kondensationspolymeren beschrieben, in dem Abfallmaterial, das während der Herstellung von Gegenständen aus den Kondensationspolymeren anfällt, einer wäßrigen Hydrolyse bei einer Temperatur im Bereich von 200 bis 300°C und einem Überdruck von mindestens 15 Atmosphären in einer speziellen Apparatur unterworfen wird. Bei dem genannten Verfahren wird die Hydrolyse mit unter hohem Druck stehendem Wasserdampf durchgeführt. Die Hydrolyse von Kondensationsprodukten, die Füllstoffe wie Glasfasern enthalten können oder von Blends wird jedoch nicht beschrieben.

In der US 3,939,153 wird ein Verfahren zur Herstellung von Caprolactam aus Polycaprolactam beschrieben, in dem eine Schmelze des Polymers und überhitzter Wasserdampf kontinuierlich bei Temperaturen von mindestens 315°C miteinander in Kontakt gebracht werden. Nachteilig an diesem Verfahren ist eine niedrige Ausbeute von maximal 20%.

Aufgabe der vorliegenden Erfindung war es daher, ein Verfahren zur Gewinnung von Caprolactam aus Polymeren, enthaltend als wiederkehrende Einheit

-[-N(H)-(CH₂)₅-C(O)-]-,

oder aus Mischungen enthaltend solche Polymeren, zur Verfügung zu stellen, das höhere Ausbeuten an Caprolactam in Abwesenheit eines Katalysators liefert. Des weiteren sollte ein Verfahren zur Verfügung gestellt werden, das es gestattet, Polycaprolactam-haltige Abfälle mit einem Gehalt an anorganischen Füllstoffen unter Gewinnung von Caprolactam zu verwerten, ohne Einbußen an der Ausbeute hinnehmen zu müssen.

Demgemäß wurde ein Verfahren zur Gewinnung von Caprolactam aus caprolactamhaltigen Polymeren in Gegenwart von überhitztem Wasser gefunden, indem man Polymere, die die wiederkehrende Einheit

-[-N(H)-(CH₂)₅-C(O)-]-

enthalten, oder Mischungen, bestehend im wesentlichen aus

| | |
|---|---|
| 40 bis 99,99 Gew.-% | eines Polymeren mit der wiederkehrenden Einheit -[-N(H)-(CH₂)₅-C(O)-]- |
| 0,01 bis 50 Gew.-% | Zusatzstoffen, ausgewählt aus der Gruppe, bestehend aus anorganischen Füllstoffen, organischen und anorganischen Pigmenten und Farbstoffen, |
| 0 bis 10 Gew.-% | organische und/oder anorganische Additive, |
| 0 bis 40 Gew.-% | nicht-polyamidhaltige Polymere, |
| 0 bis 60 Gew.-% | Polyamide, mit der Ausnahme von Polycaprolactam und Copolyamiden, hergestellt auf der Basis von Caprolactam, |

mit überhitztem Wasser mit einer Temperatur im Bereich von 280 bis 320 °C und einem Druck im Bereich von 7,5 bis 15 MPa, einem Gewichtsverhältnis von Wasser zu Polymer mit der wiederkehrenden Einheit -[-N(H)-(CH₂)₅-C(O)-]- von 5:1 bis 13:1 und einer Reaktionszeit von unter 3 h in Kontakt bringt, mit der Maßgabe, daß die Reaktionsmischung, bestehend im wesentlichen aus Wasser und dem eingesetzten Polymeren oder der eingesetzten Mischung, unter den Bedingungen der Hydrolyse keine gasförmige Phase enthält.

Des weiteren wurde ein Vorrichtung zur Durchführung des erfindungsgemäßen Verfahrens sowie die Verwendung des erfindungsgemäßen Verfahrens und der erfindungsgemäßen Vorrichtung zum Recycling von Polycaprolactam-haltigen Abfällen gefunden.

Erfindungsgemäß geht man von Polymeren aus, die die wiederkehrende Einheit

-[-N(H)-(CH₂)₅-C(O)-]-

enthalten, oder von Mischungen, bestehend im wesentlichen aus

| | |
|---|---|
| 40 bis 99,99, | vorzugsweise von 70 bis 90 Gew.-% eines Polymeren mit der wiederkehrenden Einheit -[-N(H)-(CH₂)₅-C(O)-]- |
| 0,01 bis 50, | vorzugsweise von 4 bis 10 Gew.-% Zusatzstoffen, ausgewählt aus der Gruppe, bestehend aus anorganischen Füllstoffen, organischen und anorganischen Pigmenten und Farbstoffen, |
| 0 bis 10, | vorzugsweise von 0,1 bis 5 Gew.-% organische und/oder anorganische Additive, |
| 0 bis 40, | vorzugsweise von 5 bis 25 Gew.-% nicht-polyamidhaltige Polymere, |
| 0 bis 60, | vorzugsweise von 10 bis 30 Gew.-% Polyamide, mit der Ausnahme von Polycaprolactam und Copolyamiden, hergestellt auf der Basis von Caprolactam. |

Bevorzugt setzt man als Polymer Polycaprolactam mit einer relativen Viskosität im Bereich von 1 bis 10, vorzugsweise von 2,0 bis 4,0 (gemessen bei einer Konzentration von 1 g Polymer pro 100 ml in 96 gew.-%iger Schwefelsäure bei 25°C) ein. Man kann ferner auch Polycaprolactam einsetzen, das Oligomere im Bereich von 0,01 bis 10, bevorzugt von 1 bis 5 Gew.-%, bezogen auf die Gesamtmenge, enthält. Prinzipiell kann man das erfindungsgemäße Verfahren auch ausführen, wenn man anstelle von Polycaprolactam Oligomere von Caprolactam einsetzt.

Des weiteren kann man auch Copolyamide aus Caprolactam und anderen polyamidbildenden Monomeren, beispielsweise Salze, gebildet aus einer Dicarbonsäure wie Adipinsäure, Sebacinsäure und Terephthalsäure und einem Diamin wie Hexamethylendiamin und Tetramethylendiamin, vorzugsweise AH-Salz (aus Adipinsäure und Hexamethylendiamin), und Lactame wie Laurinlactam einsetzen.

Nach bisherigen Beobachtungen können alle bekannten Polycaprolactame nach dem erfindungsgemäßen Verfahren zu Caprolactam umgesetzt werden, beispielsweise auch ein Polycaprolactam, das in Gegenwart von Mono- oder Dicarbonsäuren oder Aminen, die als Kettenregler wirken, hergestellt wurde, wie Essigsäure, Propionsäure, Benzoesäure,
C₄-C₁₀-Alkandicarbonsäuren wie Adipinsäure, Pimelinsäure, Korksäure, Azelainsäure, Sebazinsäure, Undecandisäure, Dodecandisäure und deren Mischungen,
C₅-C₈-Cycloalkandicarbonsäuren wie Cyclopentan-1,3-dicarbonsäure, Cyclohexan-1,4-dicarbonsäure und deren Mischungen,

Benzol- und Naphthalindicarbonsäuren, die bis zu zwei Sulfonsäuregruppen, wobei hierunter auch die entsprechenden Alkalimetallsalze mitzuzählen sind, tragen können und deren Carbonsäuregruppen nicht benachbart sind, wie Terephthalsäure, Isophthalsäure, Naphthalin-2,6-dicarbonsäure, 5-Sulphoisophthalsäure sowie deren Natrium- und Lithiumsalz, und deren Mischungen, und 1,4-Piperazin-di(C₁-C₆-alkandicarbonsäuren) wie 1,4-Piperazindiessigsäure, 1,4-Piperazin-dipropionsäure, 1,4-Piperazin-dibutansäure, 1,4-Piperazin-dipentansäure, 1,4-Piperazin-dihexansäure.

Entsprechende Copolyamide sind dem Fachmann bekannt und nach Verfahren herstellbar, die beispielsweise in WO 93/25736, DE-A 14 95 198 und DE-A 25 58 480 beschrieben sind.

Als anorganische Füllstoffe kann man nach bisherigen Beobachtungen alle in der Konfektionierung von Polyamiden gebräuchlichen Füllstoffe wie Glasfasern, Calciumcarbonat und Talkum einsetzen. Als anorganische und organische Pigmente und Farbstoffe kann man nach bisherigen Beobachtungen alle zum Färben von Polyamiden gebräuchlichen Pigmente und Farbstoffe wie Titandioxid, Cadmiumsulfid, Eisenoxide oder Farbruße sowie die üblichen Spinnfarbstoffe wie Chrom- oder Kupferkomplexverbindungen, einsetzen.

Als organische und anorganische Additive kann man die üblichen Stabilisatoren und Oxidationsverzögerer, Mittel gegen Wärmezersetzung und Zersetzung gegen ultraviolettes Licht, Antistatika und Flammschutzmittel einsetzen.

Oxidationsverzögerer und Wärmestabilisatoren sind beispielsweise sterisch gehinderte Phenole, Hydrochinone, Phosphite und Abkömmlinge und substituierte Vertreter dieser Gruppe und Mischungen dieser Verbindungen sowie Kupferverbindungen wie Kupfer-(I)-iodid und Kupfer-(II)-acetat.

Beispiele für UV-Stabilisatoren sind substituierte Resorcine, Salicylate, Benzotriazole, Benzophenone und Verbindungen des HALS-(hindered amine light stabilizer)-Typs, ferner eignen sich hierfür Mangan-(II)-Verbindungen.

Als Antistatika kann man die üblichen Stoffe, beispielsweise Polyalkylenoxide und Derivate davon einsetzen.

Als Flammschutzmittel kann man die üblichen phosphor- und stickstoff-phosphorhaltigen Verbindungen einsetzen wie Ester der Phosphorsäure, phosphorigen Säure und von Phosphon- und Phosphinsäure sowie tertiäre Phosphine und Phosphinoxide wie Triphenylphosphinoxid, Phosphonnitrilchlorid, Phosphorsäureesteramide, Phosphorsäureamide, Phosphinsäureamide, Tris-(aziridinyl)-phosphinoxid und Tetrakis(hydroxymethyl)phosphoniumchlorid.

Als nicht-polyamidhaltige Polymere kann man die üblichen thermoplastischen, technischen Polymere einsetzen wie Polymere auf der Basis von Ethylen, Propylen, Styrol sowie dessen Copolymere mit Butadien und Acrylnitril (ABS-Kunststoffe).

Als Polyamide, mit der Ausnahme von Polycaprolactam und Copolyamiden hergestellt auf der Basis von Caprolactam, kann man beispielsweise Polyamid 66, Polyamid 610 und Polyamid 46 einsetzen.

Bevorzugt geht man Polycaprolactam mit einem Gehalt an anorganischen Füllstoffen, insbesondere Glasfasern, aus, das entsorgt werden soll oder von Abfällen, die bei der Polycaprolactamherstellung und dessen Verarbeitung zu Fäden, Folien, Spritzguß-oder Extrusionsteilen anfallen, ferner geformte Gebrauchsgegenstände wie Folien, Verpackungen, Gewebe, Teppichbodenfasern, Fäden und extrudierte Teile, die entsorgt werden sollen.

Erfindungsgemäß bringt man die oben genannten Polymeren oder Mischungen mit überhitztem Wasser, das eine Temperatur im Bereich von 280 bis 320, vorzugsweise von 295 bis 310, besonders bevorzugt von 300 bis 305°C und einen Druck im Bereich von 7,5 bis 15, vorzugsweise von 10 bis 15 MPa, besonders bevorzugt von 10 bis 12 MPa aufweist, in Kontakt, wobei man das Gewichtsverhältnis von Wasser zu dem Polymer mit der wiederkehrenden Einheit -[-N(H)-(CH₂)₅-C(O)-]- im Bereich von 5:1 bis 13:1, vorzugsweise von 8:1 bis 13:1, wählt. Des weiteren wählt man erfindungsgemäß die Reaktionszeit unter 3 h, bevorzugt im Bereich von 15 bis 90 min, besonders bevorzugt von 30 bis 60 min.

Wesentlich für den Erfolg des erfindungsgemäßen Verfahrens ist es, daß man die Bedingungen der Hydrolyse innerhalb der angegebenen Werte so wählt, daß die Reaktionsmischung, bestehend im wesentlichen aus Wasser und dem eingesetzten Polymer, oder der eingesetzten Mischung, keine gasförmige Phase enthält. Nach bisherigen Beobachtungen führen gasförmige Anteile in der Reaktionsmischung zu Ausbeuteverlusten.

Die nach der Hydrolyse erhaltene Reaktionsmischung kann man wie üblich aufarbeiten, beispielsweise indem man Caprolactam von gegebenenfalls vorhandenen Feststoffen wie Glasfasern, Füllstoffen, Pigmenten usw. trennt und einer weiteren Reinigungsstufe, bevorzugt einer Destillation, zuführt.

Das nach dem erfindungsgemäßen Verfahren gewonnene Caprolactam läßt sich natürlich wieder zu Polycaprolactam oder entsprechenden Copolymeren und Blends verarbeiten.

In einer bevorzugten Ausführungsform schmilzt man die oben genannten Polymere oder Mischungen auf, indem man sie auf eine Temperatur im Bereich von 250 bis 350°C, vorzugsweise von 290 bis 300°C erhitzt. Anschließend oder bevorzugt gleichzeitig verdichtet man die so erhaltene Schmelze auf einen Druck im Bereich von 7,5 bis 30, vorzugsweise von 10 bis 15 MPa, wobei man zweckmäßig den Druck so wählt, daß er geringfügig über dem Druck des überhitzten Wassers liegt, mit dem die Schmelze (Schmelze A) anschließend in Kontakt gebracht wird, um so eine Rückströmung des überhitzten Wassers in die Aufschmelzvorrichtung (1) zu vermeiden. In einer besonders bevorzugten Ausführungsform führt man den Schmelzvorgang und das Verdichten gleichzeitig auf einem üblichen Extruder als Aufschmelzvorrichtung (1) durch.

Anschließend bringt man das überhitzte Wasser und die in der Aufschmelzvorrichtung (1) verdichtete Schmelze (Schmelze A) in einem Hydrolysereaktor (2) in Kontakt. Die Temperaturen und den Druckbereich wählt man erfindungsgemäß so, daß keine gasförmige Phase insbesondere während der Hydrolyse im Hydrolysereaktor vorliegt. Dabei liegen die Temperaturen im allgemeinen im Bereich von 280 bis 320, vorzugsweise von 290 bis 310, besonders bevorzugt von 300 bis 305°C, und der Druck in der Regel im Bereich von 7,5 bis 15, vorzugsweise von 10 bis 12 MPa. Die Verweilzeit im Hydrolysereaktor hängt im wesentlichen von der zugesetzten Menge an Wasser ab, bezogen auf die wiederkehrende Einheit -[N(H)- (CH₂)₅-C(O)-]-, und liegt üblicherweise im Bereich von 15 bis 90, vorzugsweise von 30 bis 60 min.

Als Hydrolysereaktor (2) kann man druckfeste Rohre verwenden, Wobei der Hydrolysereaktor mit oder ohne Einbauten wie Mischelemente des Typs SMX der Fa. Sulzer versehen werden kann (s. Chem.-Ing.-Tech. 62 (1990) 650-654). In einer bevorzugten Ausführungsform setzt man einen Rohrreaktor mit einem L/D-Verhältnis im Bereich von 20:1 bis 150:1, bevorzugt von 50:1 bis 120:1 ein.

Den Austrag des Hydrolysereaktors führt man in einer bevorzugten Ausführungsform in eine Entspannungsvorrichtung (3), die ein-oder zweistufig sein kann, wobei sich durch den Druckabfall auf einen Bereich von 0,1 bis 1,6, vorzugsweise von 0,1 bis 0,4 kPa, im wesentlichen zwei Phasen bilden: eine gasförmige Phase B, enthaltend im wesentlichen Wasser und gegebenenfalls geringe Mengen an anderen flüchtigen Stoffen wie Caprolactam und Spuren flüchtiger Aminverbindungen und eine nichtgasförmige Phase C, enthaltend im wesentlichen die Hauptmenge an gespaltenem Caprolactam, sowie, je nach eingesetzter Mischung, gegebenenfalls Zusatzstoffe wie Glasfasern, Pigmente, Additive etc. Üblicherweise trennt man die gasförmige und wasserdampfhaltige Phase B von der nichtgasförmigen Phase C in der Entspannungsvorrichtung (3) ab, wobei man bevorzugt in einer dafür geeigneten Apparatur, beispielsweise einer Destillationsapparatur oder einer Verdampferstufe, das Wasser abtrennt und dann dem Wasser beimischt, das zur Hydrolyse der Polymeren oder Mischungen in den Hydrolysereaktor (2) eingebracht wird.

Die nichtgasförmige Phase C, die in der Regel Wasser, gegebenenfalls Zusatzstoffe, organische und anorganische Additive, nicht-polyamidhaltige Polymere und Polyamide, mit Ausnahme von Polycaprolactam und Copolyamiden, hergestellt auf der Basis von Caprolactam, und in der Regel 5 bis 20 Gew.-% Caprolactam enthält, führt man in einer bevorzugten Ausführungsform einer Abtrennvorrichtung (4) zu, in der gegebenenfalls vorkommende nichtlösliche Anteile wie Zusatzstoffe, beispielsweise Glasfasern, Pigmente andere Polymere usw. entfernt werden.

Als Abtrennvorrichtung (4) kann man eine übliche Filtervorrichtung wie ein Bandfilter und ein rückspülbares Kerzenfilter oder andere übliche Apparate, die einen kontinuierlichen oder periodischen Austrag ermöglichen, verwenden, bevorzugt ein Bandfilter oder ein rückspülbares Kerzenfilter.

Die von unlöslichen Bestandteilen befreite Lösung kann man nach an sich bekannten Methoden aufarbeiten, beispielsweise indem man das Wasser destillativ von Caprolactam trennt und analog zur Aufarbeitung der gasförmigen Phase B dem Hydrolysewasser zuführt, und das Caprolactam einer Reinigungsstufe, beispielsweise der Reinigungstufe für Rohcaprolactam in einer bestehenden Caprolactam-Anlage, zuführt. Weitere Möglichkeiten wie das erfindungsgemäß erhaltene Caprolactam, so gewünscht, gereinigt werden kann, sind beispielsweise aus den EP-A 568,882 und 570,843 bekannt. Das gereinigte Caprolactam steht dann im allgemeinen für eine weitere Verwendung, insbesondere zur Herstellung von PA 6, zur Verfügung.

Das erfindungsgemäße Verfahren verwendet man erfindungsgemäß zum Recycling von Polycaprolactam-haltigen Abfällen wie gebrauchten Teppichen, Teppichresten, Polyamid-6-Produktionsabfällen und Polyamid-Gemischen, die bis zu 60 Gew.-% Polyamide, die nicht auf Basis von Caprolactam hergestellt wurden, enthalten können.

Die Vorteile des erfindungsgemäßen Verfahrens gegenüber Verfahren aus dem Stand der Technik sind die Spaltausbeuten von bis zu 96 %, kurze Verweilzeiten und geringere Mengen an behandlungsbedürftigen und zu entsorgenden Abfällen und Lösungsmitteln.

### Beispiele

### Beispiel 1

Einem 3-1-Rohrreaktor (Länge/Durchmesser-Verhältnis: 110:1) wurden stündlich 0,3 kg Polycaprolactam (Ultramid® BS 700, mit einer relativen Viskosität von 2,7, gemessen an einer 1 gew.-%igen Lösung in 96 gew.-%iger Schwefelsäure bei einer Temperatur von 23°C) mit einer Temperatur von 270°C und einem Druck von 20 MPa sowie 2,7 kg Wasser mit einer Temperatur von 290°C und einem Druck von 12 MPa zugeführt. Die durchschnittliche Verweilzeit betrug 60 min. Nach Verlassen des Reaktors wurde auf eine Temperatur von 115°C gekühlt und auf einen Druck von 0,1 kPa entspannt. Die erhaltene Reaktionsmischung wurde gaschromatographisch analysiert. Die Ergebnisse sind in der untenstehenden Tabelle aufgeführt.

### Beispiele 2 bis 11

Beispiel 1 wurde bei unterschiedlichen Wasser zu Polymer-Verhältnissen und unterschiedlichen Temperaturen wiederholt. Die Ergebnisse sind in der folgenden Tabelle zusammengefaßt.

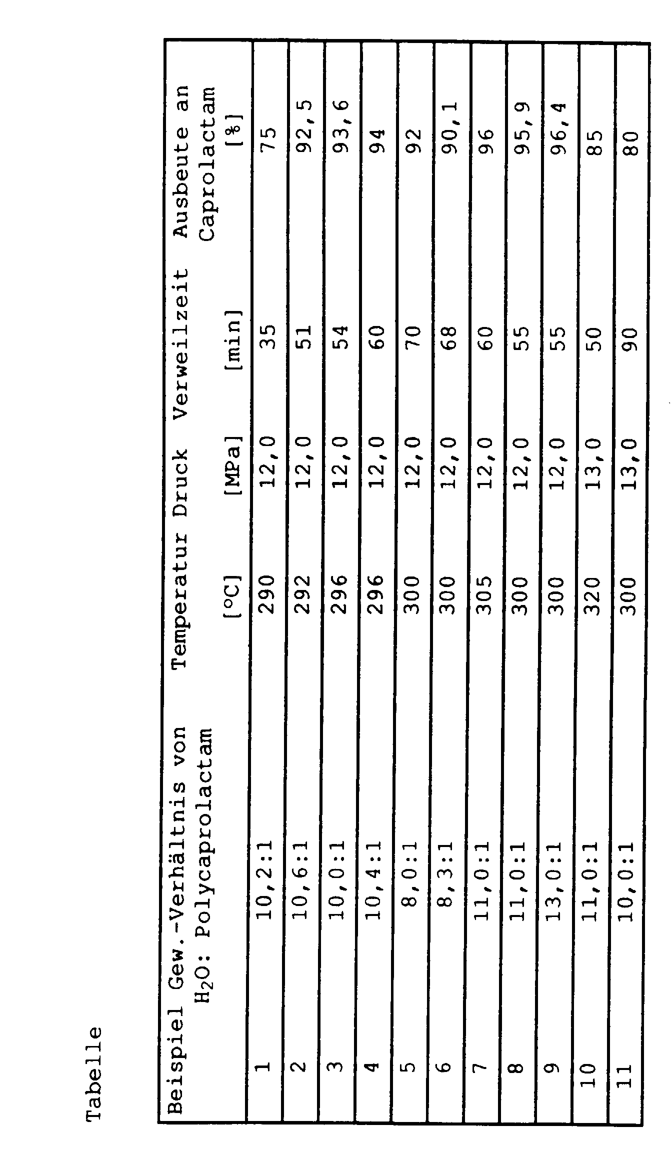

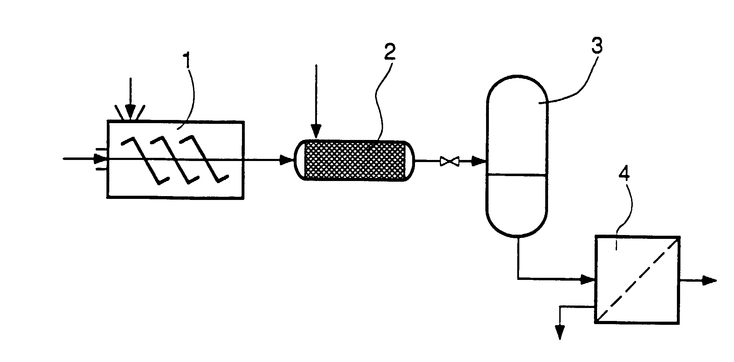

## Patentansprüche

1. Verfahren zur Gewinnung von Caprolactam aus caprolactamhaltigen Polymeren in Gegenwart von überhitztem Wasser, dadurch gekennzeichnet, daß man Polymere, die die wiederkehrende Einheit
-[-N(H)-(CH₂)₅-C(O)-]-
enthalten, oder Mischungen, bestehend im wesentlichen aus 40 bis 99,99 Gew.-% eines Polymeren mit der wiederkehrenden Einheit
-[-N(H)-(CH₂)₅-C(O)-]-
| | |
|---|---|
| 0,01 bis 50 Gew.-% | Zusatzstoffen, ausgewählt aus der Gruppe, bestehend aus anorganischen Füllstoffen, organischen und anorganischen Pigmenten und Farbstoffen, |
| 0 bis 10 Gew.-% | organische und/oder anorganische Additive, |
| 0 bis 40 Gew.-% | nicht-polyamidhaltige Polymere, |
| 0 bis 60 Gew.-% | Polyamide, mit der Ausnahme von Polycaprolactam und Copolyamiden, hergestellt auf der Basis von Caprolactam, |
mit überhitztem Wasser mit einer Temperatur im Bereich von 280 bis 320°C und einem Druck im Bereich von 7,5 bis 15 MPa, einem Gewichtsverhältnis von Wasser zu Polymer mit der wiederkehrenden Einheit-[-N(H)-(CH₂)₅-C(O)-]- von 5:1 bis 13:1 und einer Reaktionszeit von unter 3 h in Kontakt bringt, mit der Maßgabe, daß die Reaktionsmischung, bestehend im wesentlichen aus Wasser und dem eingesetzten Polymeren oder der eingesetzten Mischung, unter den Bedingungen der Hydrolyse keine gasförmige Phase enthält.

2. Verfahren gemäß Anspruch 1, dadurch gekennzeichnet, daß man folgende Schritte durchführt:
(a) Aufschmelzen und Verdichten des Polymeren oder der Mischung auf eine Temperatur im Bereich von 250 bis 350°C und einen Druck im Bereich von 7,5 bis 30 MPa unter Erhalt einer Schmelze A,
(b) Mischen von Wasser mit einer Temperatur im Bereich von 280 bis 320°C und einem Druck im Bereich von 7,5 bis 15 MPa mit der Schmelze A in einem Hydrolysereaktor,
(c) Austragen einer den Hydrolysereaktor verlassenden nichtgasförmigen Phase unter Entspannungsverdampfung unter Erhalt einer gasförmigen Phase B und einer nichtgasförmigen Phase C,
(d) Abtrennen von gegebenenfalls nichtflüssigen Anteilen in der nichtgasförmigen Phase C unter Erhalt einer flüssigen Phase D, enthaltend Caprolactam, und einer festen Phase E, und
(e) gewünschtenfalls Überführen der in Stufe d) erhaltenen flüssigen Phase D in eine Reinigungsstufe für Caprolactam.

3. Vorrichtung zur Gewinnung von Caprolactam aus Polymeren, die die wiederkehrende Einheit
-[-N(H)-(CH₂)₅-C(O)-]-
enthalten, oder Mischungen, bestehend im wesentlichen aus 40 bis 99,99 Gew.-% eines Polymeren mit der wiederkehrenden Einheit
-[-N(H)-(CH₂)₅-C(O)-]-
| | |
|---|---|
| 0,01 bis 50 Gew.-% | Zusatzstoffen, ausgewählt aus der Gruppe, bestehend aus anorganischen Füllstoffen, organischen und anorganischen Pigmenten und Farbstoffen, |
| 0 bis 10 Gew.-% | organische und/oder anorganische Additive, |
| 0 bis 40 Gew.-% | nicht-polyamidhaltige Polymere, |
| 0 bis 60 Gew.-% | Polyamide, mit der Ausnahme von Polycaprolactam und Copolyamiden, hergestellt auf der Basis von Caprolactam, |
durch Hydrolyse mit überhitztem Wasser, bestehend im wesentlichen aus einer Aufschmelzvorrichtung (1), einem damit verbundenen Hydrolysereaktor (2) zum Mischen und zur Durchführung der Hydrolyse sowie einer mit dem Hydrolysereaktor (2) verbundenen Entspannungsvorrichtung (3) und einer Abtrennvorrichtung (4).

4. Verwendung der Vorrichtung gemäß Anspruch 3 zum Recycling von polycaprolactamhaltigen Abfällen.

5. Verwendung der Verfahren gemäß den Ansprüchen 1 bis 2 zum Recycling von polycaprolactam-haltigen Abfällen.

6. Verfahren gemäß Anspruch 1, dadurch gekennzeichnet, daß man anstelle von Polymeren, die die wiederkehrende Einheit -[-N(CH)-(CH₂)₅-C(O)-]- enthalten, Oligomere, die die wiederkehrende Einheit -[-N(H)-(CH₂)₅-C(O)-]- enthalten, einsetzt.

## Claims

1. A process for obtaining caprolactam from caprolactam-containing polymers in the presence of superheated water, which comprises bringing polymers which contain the repeating unit
-[-N(H)-(CH₂)₅-C(O)-]-
or mixtures consisting essentially of from 40 to 99.99 % by weight of a polymer containing the repeating unit
-[-N(H)-(CH₂)₅-C(O)-]-
| | |
|---|---|
| from 0.01 to 50 % | by weight of additives selected from the group consisting of inorganic fillers, organic and inorganic pigments and dyes, |
| from 0 to 10 % | by weight of organic or inorganic additives, |
| from 0 to 40 % | by weight of non-polyamide-containing polymers and |
| from 0 to 60 % | by weight of polyamides, with the exception of polycaprolactam and copolyamides prepared from caprolactam, |
into contact with superheated water at from 280 to 320°C and at from 7.5 to 15 MPa and a weight ratio of water to polymer containing the repeating unit -[-N(H)-(CH₂)₅-C(O)-]- of 5:1 to 13:1 and in a reaction time of less than 3 hours, with the proviso that the reaction mixture, consisting essentially of water and the polymer used or the mixture used, contains no gaseous phase under the conditions of the hydrolysis.

2. A process as claimed in claim 1, wherein the following steps are carried out:
(a) melting and compression of the polymer or of the mixture at from 250 to 350°C and at from 7.5 to 30 MPa to give a melt A,
(b) mixing of water maintained at from 280 to 320°C and at from 7.5 to 15 MPa with the melt A in a hydrolysis reactor,
(c) discharge of a nongaseous phase leaving the hydrolysis reactor, with flash evaporation to give a gaseous phase B and a nongaseous phase C,
(d) separating off any nonliquid fractions in the nongaseous phase C to give a liquid phase D, containing caprolactam, and a solid phase E and
(e) if desired, transfer of the liquid phase D obtained in stage d) to a purification stage for caprolactam.

3. Apparatus for obtaining caprolactam from polymers which contain the repeating unit
-[-N(H)-(CH₂)₅-C(O)-]-
or mixtures consisting essentially of from 40 to 99.9 % by weight of a polymer containing the repeating unit
-[-N(H)-(CH₂)₅-C(O)-]-
| | |
|---|---|
| from 0.01 to 50 % | by weight of additives selected from the group consisting of inorganic fillers, organic and inorganic pigments and dyes, |
| from 0 to 10 % | by weight of organic or inorganic additives, |
| from 0 to 40 % | by weight of non-polyamide-containing polymers and |
| from 0 to 60 % | by weight of polyamides, with the exception of polycaprolactam and copolyamides prepared from caprolactam, |
by hydrolysis with superheated water, consisting essentially of a melting apparatus (1), a hydrolysis reactor (2) connected thereto and intended for mixing and for carrying out the hydrolysis, and a let-down apparatus (3), connected to the hydrolysis reactor (2), and a separation apparatus (4).

4. The use of an apparatus as claimed in claim 3 for recycling polycaprolactam-containing wastes.

5. The use of a process as claimed in claim 1 or 2 for recycling polycaprolactam-containing wastes.

6. A process as claimed in claim 1, wherein oligomers which contain the repeating unit -[-N(CH)-(CH₂)₅-C(O)-]- are used instead of polymers which contain the repeating unit -[-N(H)-(CH₂)₅-C(O)-]-.

## Revendications

1. Procédé de production de caprolactame à partir de polymères contenant du caprolactame, en présence d'eau surchauffée, caractérisé en ce qu'on met en contact des polymères, qui contiennent l'unité répétitive :
-[-N(H)-(CH₂)₅-C(O)-]-
ou des mélanges, constitués essentiellement
de 40 à 99,99% en poids d'un polymère comportant l'unité répétitive :
-[-N(H)-(CH₂)₅-C(O)-]-
de 0,01 à 50% en poids d'adjuvants, choisis parmi le groupe comprenant des charges inorganiques, des pigments organiques et inorganiques et des colorants,
de 0 à 10% en poids d'additifs organiques et/ou inorganiques,
de 0 à 40% en poids de polymères ne contenant pas de polyamide,
de 0 à 60% en poids de polyamides, à l'exception de polycaprolactame et de copolyamides, préparés à base de caprolactame,
avec de l'eau surchauffée à une température de l'ordre de 280 à 320°C et sous une pression de l'ordre de 7,5 à 15 MPa, un rapport pondéral entre l'eau et le polymère présentant l'unité répétitive -[-N(H)-(CH₂)₅-C(O)-]- de 5/1 à 13/1 et pendant une période réactionnelle inférieure à 3 heures, à la condition que le mélange réactionnel, constitué sensiblement d'eau et du polymère mis en oeuvre ou du mélange mis en oeuvre, ne contienne pas de phase gazeuse dans les conditions de l'hydrolyse.

2. Procédé suivant la revendication 1, caractérisé en ce qu'on effectue les étapes suivantes :
(a) faire fondre et condenser le polymère ou le mélange à une température de l'ordre de 250 à 350°C et sous une pression de l'ordre de 7,5 à 30 MPa, avec obtention d'une masse fondue A,
(b) mélanger de l'eau à une température de l'ordre de 280 à 320°C et à une pression de l'ordre de 7,5 à 15 MPa avec la masse fondue A dans un réacteur d'hydrolyse,
(c) évacuer une phase non gazeuse quittant le réacteur d'hydrolyse avec vaporisation instantanée et obtention d'une phase gazeuse B et d'une phase non gazeuse C,
(d) isoler des fractions éventuellement non liquides dans la phase non gazeuse C avec obtention d'une phase liquide D, contenant du caprolactame, et d'une phase solide E, et
(e) transférer éventuellement la phase liquide D, obtenue dans l'étape (d), dans une étape de purification du caprolactame.

3. Dispositif de production de caprolactame à partir de polymères, qui contiennent l'unité répétitive :
-[-N(H)-(CH₂)₅-C(O)-]-
ou de mélanges, constitués sensiblement
de 40 à 99,99% en poids d'un polymère comportant l'unité répétitive :
-[-N(H)-(CH₂)₅-C(O)-]-
de 0,01 à 50% en poids d'adjuvants, sélectionnés parmi le groupe comprenant des charges inorganiques, des pigments organiques et inorganiques et des colorants,
de 0 à 10% en poids d'additifs organiques et/ou inorganiques,
de 0 à 40% en poids de polymères ne contenant pas de polyamide,
de 0 à 60% en poids de polyamides, à l'exception du polycaprolactame et de copolyamides, préparés à base de caprolactame,
par hydrolyse avec de l'eau surchauffée, comprenant essentiellement un dispositif de fusion (1), un réacteur d'hydrolyse (2) relié à celui-ci et destiné au mélange et à la mise en oeuvre de l'hydrolyse, ainsi qu'un dispositif de détente (3) relié au réacteur d'hydrolyse (2) et un dispositif de séparation (4).

4. Utilisation du dispositif suivant la revendication 3, pour le recyclage de déchets contenant du polycaprolactame.

5. Utilisation du procédé suivant l'une des revendications 1 et 2, pour le recyclage de déchets contenant du polycaprolactame.

6. Procédé suivant la revendication 1, caractérisé en ce que, au lieu de polymères, qui contiennent l'unité répétitive :
-[-N(H)-(CH₂)₅-C(O)-]-
on met en oeuvre des oligomères, qui contiennent l'unité répétitive :
-[-N(H)-(CH₂)₅-C(O)-]-
